# FASCICULE DE BREVET EUROPEEN

(11) **EP 1 687 051 B1**
(45) Date de publication et mention de la délivrance du brevet: **21.08.2013**
(21) Numéro de dépôt: 04818444.4
(22) Date de dépôt: 13.10.2004
(51) Int. Cl.: A61M 5/32

(54) **DISPOSITIF D INJECTION SECURISE POUR UNE SERINGUE**
SICHERHEITSINJEKTIONSVORRICHTUNG FÜR EINE SPRITZE
SAFETY INJECTION DEVICE FOR SYRINGE

(30) Priorité: 29.10.2003 FR 0312642
(43) Date de publication de la demande: 09.08.2006
(73) Titulaire: Tech Group Europe Limited, Dublin 2 (IE)
(72) Inventeur: CHEVALLIER, Stéphane, F-77178 Saint Pathus (FR)
(74) Mandataire: Intès, Didier Gérard André
(86) Numéro de dépôt international: PCT/FR2004/002597
(87) Numéro de publication internationale: WO 2005/046772

(56) Documents cités:
- WO-A-01/41841
- FR-A- 2 830 765
- FR-A- 2 835 753
- US-A1- 2002 156 426
- US-B1- 6 613 022

## Description

La présente invention concerne un dispositif d'injection sécurisé, comprenant un fourreau de support ayant une extrémité proximale et une extrémité distale, un corps de seringue solidaire de ce fourreau de support et un fourreau de protection susceptible de coulisser axialement entre une position rentrée dans un espace annulaire ménagé entre le corps de seringue et le fourreau de support et une position sortie de protection dans laquelle il dépasse au-delà de l'extrémité distale du fourreau de support, le dispositif comprenant, en outre, des moyens de retenue aptes à adopter une configuration active de retenue pour retenir le fourreau de protection en position rentrée et, à partir de cette configuration active, à être sollicités pour permettre la sortie du fourreau de protection sous l'action de moyens de poussée.

Des dispositifs de ce type sont connus par les documents WO 01/41841 et WO 03/068298.

Dans ces dispositifs connus, le fourreau de support et le fourreau de protection sont assemblés et pré-montés, puis le corps de la seringue, en général en verre, est introduit dans cet ensemble pré-monté dans lequel il est fixé par clipsage. Cette opération de montage est donc réalisée postérieurement à l'assemblage du fourreau de support et du fourreau de protection. Elle nécessite donc un équipement spécifique, distinct de l'équipement de fabrication et d'assemblage du fourreau de support et du fourreau de protection.

Une telle opération peut être souhaitable lorsque le corps de seringue, pré-rempli du liquide à injecter et stérilisé, est introduit dans l'ensemble pré-monté comprenant le fourreau de support et le fourreau de protection. Toutefois, pour certaines applications, le corps de seringue peut être mis en place alors qu'il est vide. Avec les dispositifs connus précités, même dans ce cas, il est nécessaire de fabriquer et d'assembler le fourreau de support et le fourreau de protection de mettre en place le corps de seringue dans une étape distincte, en général sur un site différent du site de fabrication, et de remplir ce dernier avant de réaliser une injection.

La présente invention vise à simplifier ce processus, en proposant un dispositif permettant d'éviter l'étape de mise en place du corps de seringue postérieurement à la fabrication et à l'assemblage des fourreaux de support et de protection.

Ce but est atteint grâce au fait que le corps de seringue est solidaire du fourreau de support par l'intermédiaire d'une bague comprenant une paroi de raccordement qui s'étend sensiblement transversalement entre le corps de seringue et le fourreau de support, cette paroi présentant au moins une fente ayant un contour fermé à travers laquelle passent des moyens de retenue pour pouvoir être sollicités à partir du côté de la paroi de raccordement opposé à l'extrémité distale du fourreau de support, et au fait que la bague forme une seule pièce venue de moulage avec le corps de seringue.

Avec ce dispositif, l'ensemble constitué par les deux fourreaux et le corps de seringue est assemblé grâce à la présence de la bague précitée. Ainsi, le corps de seringue est mis en place en même temps que les fourreaux de support et de protection et fait partie du dispositif dès sa fabrication. Du fait de la conformation particulière de cette bague de raccordement, qui présente au moins une fente, la sollicitation des moyens de retenue du fourreau de protection qui permet le passage en configuration active peut être réalisée aisément, comme dans les demandes de brevet WO 01/41841 et WO 03/068298.

En particulier, cette sollicitation peut être réalisée par la tête du piston d'injection. Ce dernier peut être mis en place à l'intérieur du corps de seringue au moment de l'assemblage des différents éléments constitutifs du dispositif en étant poussé vers l'extrémité distale. Le remplissage du corps de seringue avec le liquide à injecter peut être réalisé postérieurement, par une traction du piston vers l'extrémité proximale du dispositif. L'aiguille de la seringue peut quant à elle être mise en place au moment de l'utilisation du dispositif. Celui-ci peut être utilisé pour un usage unique ou, éventuellement, être stérilisé entre deux utilisations. Il peut par exemple servir pour réaliser des prises de sang ou des injections réalisées avec une seringue non pré-remplie, cette dernière étant remplie juste avant l'injection.

Avantageusement, lorsque la bague constitue une pièce distincte du fourreau de support, ladite bague et ledit fourreau de support présentent chacun une jupe de fixation, ces jupes étant en contact l'une avec l'autre selon des surfaces axiales par lesquelles la bague et ledit élément sont fixés ensemble.

Avantageusement, les moyens de retenue comprennent au moins une patte de retenue qui passe à travers la fente de la paroi de raccordement et qui peut être sollicitée depuis le côté de cette paroi opposé à l'extrémité distale du fourreau de support ; pour cela, cette patte s'étend avantageusement de part et d'autre de la paroi de raccordement.

Le mode de fixation entre la bague et le fourreau de support, dont elle est distincte est avantageusement choisi parmi le soudage, l'emboîtement, l'emmanchement à force et collage.

L'invention sera bien comprise et ses avantages apparaîtront mieux à la lecture de la description détaillée qui suit, d'un mode de réalisation présenté à titre d'exemple non limitatif. La description se réfère aux dessins annexés sur lesquels :
- la figure 1 est une vue en coupe axiale du dispositif selon l'invention, avant une injection, le fourreau de protection étant dans sa position rentrée ;
- la figure 2 est une vue en coupe selon la ligne II-II de la figure 1 ;
- la figure 3 est une vue dans la même coupe que la figure 2, montrant la sollicitation des moyens de retenue du fourreau de protection en fin d'injection ;
- les figures 4 et 5 sont des vues en coupe axiale, respectivement dans les mêmes plans que la figure 1 et que la figure 2, montrant le fourreau de protection dans sa position sortie de protection ; et
- la figure 6 est une vue en perspective selon la flèche VI de la figure 1, montrant la bague de raccordement et la partie d'extrémité du corps de seringue qui est formé en une seule pièce avec cette bague.

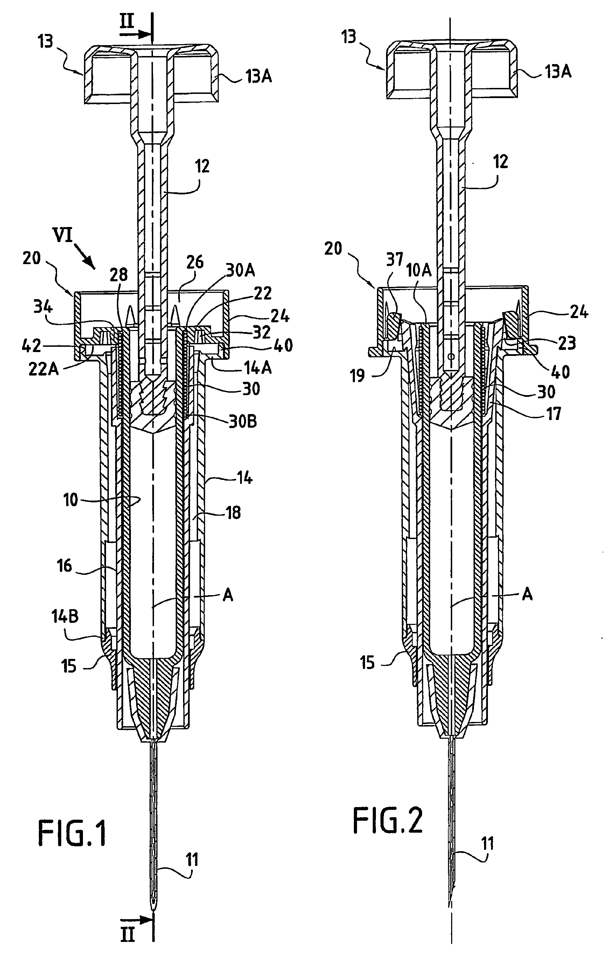

Le dispositif représenté sur les figures comprend une seringue qui a un corps de seringue 10, une aiguille 11 et un piston 12 qui peut coulisser dans ce corps pour une injection. Sur les figures 1 et 2, ce piston est reculé vers l'arrière, c'est-à-dire dans le sens allant de l'extrémité distale vers l'extrémité proximale et le dispositif est représenté avant une injection.

Ce dispositif comporte également un fourreau de support 14 et un fourreau de protection 16 qui, sur les figures 1 à 3, est dans sa position rentrée, dans laquelle il s'étend essentiellement dans un espace annulaire 18 qui est ménagé entre le corps de seringue 10 et le fourreau de support 14.

Le corps de seringue 10 est solidaire du fourreau de support 14 par l'intermédiaire d'une bague 20 qui comprend une paroi de raccordement 22 qui s'étend sensiblement transversalement entre le corps de seringue et le fourreau de support. En l'espèce, cette bague est raccordée au fourreau de support par l'extrémité proximale 14A de ce dernier. La bague 20 forme une seule pièce avec le corps de seringue 10.

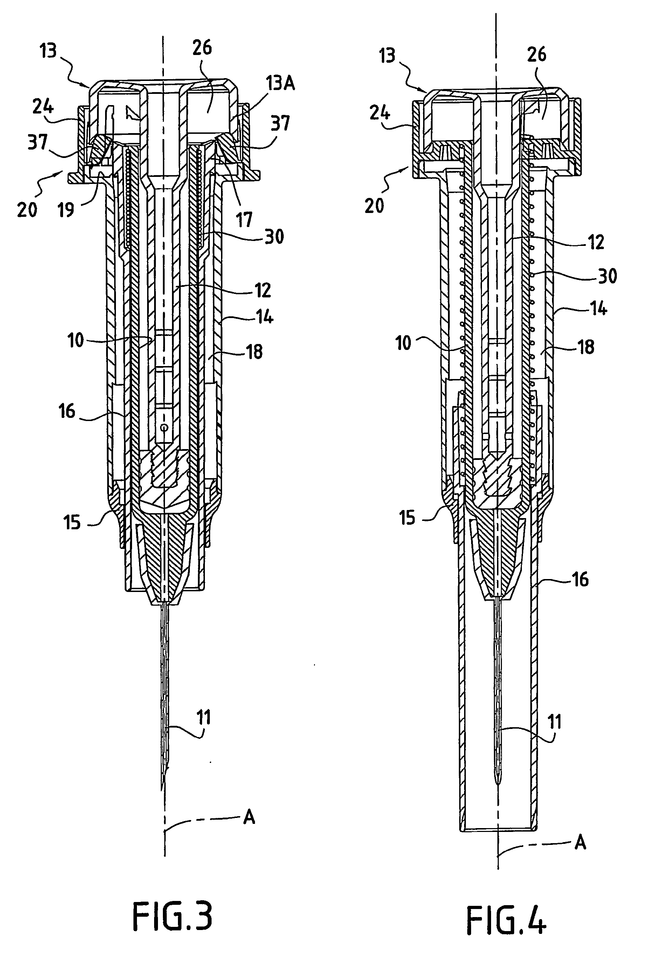

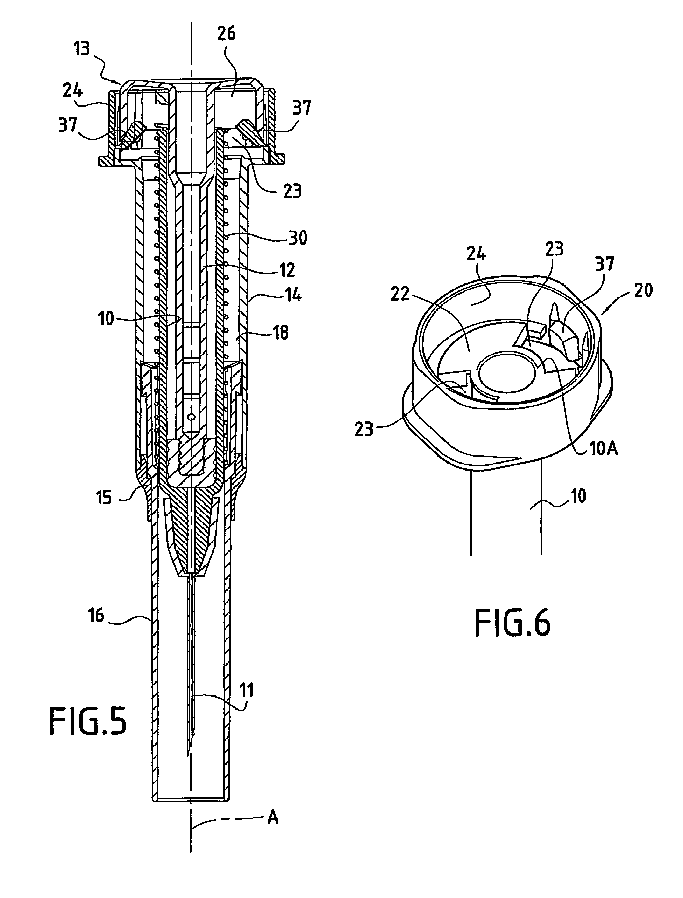

Cette bague présente une paroi axiale externe 24 qui définit, en s'étendant au-delà de la paroi de raccordement 22 dans le sens allant vers l'arrière, un logement 26 dans lequel, comme on le voit sur les figures 4 et 5, la tête d'actionnement 13 du piston vient sensiblement se loger en fin d'injection.

La paroi de raccordement 22 de la bague 20 présente, sur sa face intérieure 22A tournée vers l'extrémité distale 14B du fourreau de support 14, un renfoncement 28 dans lequel est disposée l'extrémité proximale 30A d'un ressort de poussée 30 dont l'extrémité distale 30B est en appui contre le fourreau de protection 16.

A cet effet, la paroi du fourreau de protection présente un décrochement ménageant un épaulement intérieur.

Le renfoncement 28 est en l'espèce formé par une rainure annulaire qui permet de caler axialement et radialement l'extrémité 30A du ressort 30.

La paroi de raccordement 22 présente également une autre rainure annulaire 32 qui permet d'augmenter l'épaisseur apparente de cette paroi et de préserver sa résistance mécanique tout en conservant, autant que possible, une épaisseur de matériau sensiblement constante de manière à permettre un refroidissement homogène lors du démoulage. La paroi de raccordement 22 présente au moins une nervure de renforcement qui, en l'espèce, est formée par la nervure 34 ménagée entre les rainures annulaires 28 et 32.

Dans sa position rentrée, le fourreau de protection 16 est retenu par rapport au fourreau de support 14 par des moyens de retenue qui comprennent au moins une patte de retenue qui est solidaire du fourreau de protection et le dispositif comporte au moins une surface de retenue pour cette patte, qui est fixe par rapport au corps de seringue 10, la patte de retenue étant retenue sur la surface de retenue lorsque cette patte est dans sa configuration active de retenue et étant susceptible d'être déplacée pour échapper à cette surface. En l'espèce, deux pattes de retenue diamétralement opposées 17 sont présentes, et les deux surfaces de retenue sont formées par deux zones diamétralement opposées d'un épaulement 19.

Les pattes de retenue 17 sont situées à l'extrémité proximale du fourreau de protection 16. Ces pattes sont élastiques et ont naturellement tendance à s'écarter de l'axe A du dispositif pour s'accrocher sur l'épaulement intérieur 19, tourné vers l'arrière du fourreau de support. Dans cette position, elles empêchent évidemment le fourreau de protection 16 de se déplacer vers l'avant, c'est-à-dire vers l'extrémité distale du fourreau de support.

Pour permettre la sollicitation des pattes 17 à partir du côté de la paroi de raccordement 22 qui est opposée à l'extrémité distale du fourreau de support, cette paroi 22 présente, comme on le voit mieux sur la figure 6, deux fentes 23 diamétralement opposées. C'est à travers ces fentes, qui ont un contour fermé, que les extrémités proximales des pattes 17 peuvent être sollicitées pour rabattre ces pattes vers l'axe A et les décrocher ainsi de l'épaulement 19 de manière à permettre la sortie du fourreau de protection sous l'effet de la poussée exercée par le ressort 30, de sorte que ce fourreau parvient dans sa position sortie de protection représentée sur les figures 4 et 5.

Le piston d'injection 12 est solidaire d'un organe de déclenchement qui est apte, en fin de course d'injection du piston, à déclencher la sollicitation des moyens de retenue en dehors de leur configuration active. En l'espèce, cet organe de déclenchement est formé par une jupe 13A de la tête 13 du piston.

La bague 20 présente au moins une patte de transmission qui est apte à être déplacée par cet organe de déclenchement pour solliciter les moyens de retenue en dehors de leur configuration active.

En l'espèce, la bague 20 comporte deux pattes de transmission 37 diamétralement opposées qui sont disposées au voisinage des fentes 23 de la paroi 22 précédemment évoquées. Comme on le voit sur les figures 2 et 6, les pattes de transmission 37 occupent naturellement une position dans laquelle elles s'étendent sensiblement axialement vers l'arrière de la bague 20.

Ainsi, comme le montre la figure 3, la jupe 13A de la tête 13 du piston parvient au contact de ces pattes de transmission 37 en fin d'injection, et elle tend à les rabattre vers l'axe A du dispositif. Ces pattes 37 repoussent alors les pattes de retenue 17 pour les décrocher de l'épaulement 19.

Lorsqu'elles sont repoussées vers l'axe A, les pattes 37 s'étendent en effet sensiblement en travers des fentes 23.

Dans l'exemple de réalisation représenté sur les dessins, la bague 20 est formée en une seule pièce avec le corps de seringue 10 et la paroi de raccordement 22 est raccordée à l'extrémité proximale libre 10A de ce corps 10. En revanche, la bague 20 constitue une pièce distincte du fourreau de support 14. A son extrémité proximale 14A, le fourreau 14 présente une jupe de fixation 40 qui s'étend sensiblement axialement vers l'arrière. De son côté, la bague 20 présente une jupe de fixation 42 qui s'étend vers l'avant, à partir de la paroi de raccordement 22. Ces jupes sont en contact l'une avec l'autre selon des surfaces axiales par lesquelles la bague et le fourreau de support sont fixés ensemble. En l'espèce, la jupe 42 est disposée à l'extérieur de la jupe 40. Les surfaces axiales en contact sont donc la surface axiale intérieure de la jupe 42 et la surface axiale extérieure de la jupe 40. Une disposition inverse pourrait toutefois être adoptée.

Du fait de cette disposition, la surface de contact entre, d'une part, la première pièce constituée par le corps de seringue 10 et la bague 20 et, d'autre part, la deuxième pièce constituée par le fourreau de support 14 est suffisamment importante pour mettre une fixation sécurisée de ces deux pièces. Notamment, une fixation par emmanchement à force peut être utilisée. Cette fixation peut éventuellement être sécurisée par un soudage ou un collage si nécessaire. Une fixation par emboîtement, comportant éventuellement un encliquetage, peut également être utilisée, auquel cas les surfaces axiales en contact peuvent présenter respectivement un élément saillant tel qu'une nervure et un élément rentrant tel qu'une rainure coopérant ensemble par encliquetage.

Avec l'invention, le corps de seringue 10 et la bague 20 peuvent être formés en une seule pièce par moulage ou par injection, et le fourreau de protection peut également être formé par moulage ou par injection, avant le raccordement de ces deux pièces. En l'espèce, on constate que l'extrémité distale 14B du fourreau de support 14 porte une pièce d'extrémité 15 qui ferme l'espace annulaire 18 du côté de l'extrémité distale du dispositif. Cette pièce peut être rapportée à l'extrémité du fourreau de support 14 et peut être fixée, par exemple par emmanchement à force, par soudage, par collage ou par emboîtement.

Selon une variante, on pourrait prévoir de fabriquer en une seule pièce le corps de seringue 10, la bague 20 et le fourreau de support 14 (sans sa pièce d'extrémité 15), par exemple par moulage par injection. Dans ce cas, au moins un insert mobile ayant la forme d'un tube peut être disposé dans l'espace annulaire 18 pour en préserver la forme lors du moulage.

## Revendications

1. Dispositif d'injection sécurisé, comprenant un fourreau de support (14) ayant une extrémité proximale (14A) et une extrémité distale (14B), un corps de seringue (10) solidaire de ce fourreau de support et un fourreau de protection (16) susceptible de coulisser axialement entre une position rentrée dans un espace annulaire (18) ménagé entre le corps de seringue (10) et le fourreau de support (14) et une position sortie de protection dans laquelle il dépasse au-delà de l'extrémité distale (14B) du fourreau de support, le dispositif comprenant, en outre, des moyens de retenue aptes à adopter une configuration active de retenue pour retenir le fourreau de protection en position rentrée et, à partir de cette configuration active, à être sollicités pour permettre la sortie du fourreau de protection sous l'action de moyens de poussée (30),
le corps de seringue est solidaire du fourreau de support par l'intermédiaire d'une bague (20) comprenant une paroi de raccordement (22) qui s'étend sensiblement transversalement entre le corps de seringue (10) et le fourreau de support (14), cette paroi présentant au moins une fente (23) ayant un contour fermé à travers laquelle passent des moyens de retenue pour pouvoir être sollicités à partir du côté de la paroi de raccordement (22) opposé à l'extrémité distale du fourreau de support, **caractérisé en ce que** la bague (20) forme une seule pièce venue de moulage avec le corps de seringue (10).

2. Dispositif selon la revendication 1, **caractérisé en ce que** la paroi de raccordement (22) présente, sur sa face intérieure tournée vers l'extrémité distale (14B) du fourreau de support (14), un renfoncement dans lequel est disposée l'extrémité proximale (30A) d'un ressort de poussée (30) dont l'extrémité distale est en appui contre le fourreau de protection.

3. Dispositif selon la revendication 1 ou 2, **caractérisé en ce que** la paroi de raccordement (22) présente au moins une nervure de renforcement (34).

4. Dispositif selon l'une quelconque des revendications 1 à 3, **caractérisé en ce que** la bague (20) constitue une pièce distincte du fourreau de support (14) et **en ce que** la bague (20) et ledit fourreau de support (14) présentent chacun une jupe de fixation (40), ces jupes étant en contact l'une avec l'autre selon des surfaces axiales par lesquelles la bague et ledit élément sont fixés ensemble.

5. Dispositif selon l'une quelconque des revendications 1 à 4, **caractérisé en ce que** la bague (20) constitue une pièce distincte du fourreau de support (14) et est fixée audit fourreau de support par un mode de fixation choisi parmi le soudage, l'emboîtement, l'emmanchement à force et le collage.

6. Dispositif selon l'une quelconque des revendications 1 à 5, **caractérisé en ce que** les moyens de retenue comprennent au moins une patte de retenue (17) qui passe à travers la fente (23) et qui s'étend de part et d'autre de la paroi de raccordement (22).

7. Dispositif selon l'une quelconque des revendications 1 à 6, **caractérisé en ce que** les moyens de retenue comprennent au moins une patte de retenue qui est solidaire du fourreau de protection et **en ce qu'**il comporte au moins une surface de retenue (19) pour cette patte, qui est fixe par rapport au corps de seringue (10), la patte de retenue étant retenue sur la surface de retenue (19) lorsque ladite patte est dans sa configuration active de retenue et étant susceptible d'être déplacée pour échapper à ladite surface.

8. Dispositif selon l'une quelconque des revendications 1 à 7, comportant, en outre, un piston d'injection (12), **caractérisé en ce que** le piston est solidaire d'un organe de déclenchement (13A) apte, en fin de course d'injection du piston, à déclencher la sollicitation des moyens de retenue en dehors de leur configuration active.

9. Dispositif selon la revendication 8, **caractérisé en ce que** la bague (20) présente au moins une patte de transmission (37) apte à être déplacée par l'organe de déclenchement (13A) pour solliciter les moyens de retenue en dehors de leur configuration active.

## Patentansprüche

1. Gesicherte Injektionsvorrichtung, umfassend eine Traghülse (14) mit einem proximalen Ende (14A) und einem distalen Ende (14B), einen Spritzenkörper (10), der mit dieser Traghülse fest verbunden ist, sowie eine Schutzhülse (16), die zwischen einer in einen zwischen dem Spritzenkörper (10) und der Traghülse (14) ausgebildeten ringförmigen Raum (18) eingezogenen Stellung und einer ausgefahrenen Schutzstellung, in der sie über das distale Ende (14B) der Traghülse vorsteht, axial verschieblich ist, wobei die Vorrichtung ferner Haltemittel umfaßt, die geeignet sind, eine aktive Halteausführung einzunehmen, um die Schutzhülse in eingezogener Stellung zu halten, und von dieser aktiven Ausführung ausgehend belastet zu werden, um das Ausfahren der Schutzhülse unter der Wirkung von Schubmitteln (30) zu ermöglichen, der Spritzenkörper ist mittels eines Rings (20), der eine Verbindungswand (22) umfaßt, die im wesentlichen quer zwischen dem Spritzenkörper (10) und der Traghülse (14) verläuft, mit der Traghülse fest verbunden, wobei diese Wand wenigstens einen Schlitz (23) mit einer geschlossenen Kontur aufweist, durch den hindurch Haltemittel verlaufen, um von der dem distalen Ende der Traghülse gegenüberliegenden Seite der Verbindungswand (22) belastet zu werden,
**dadurch gekennzeichnet, daß** der Ring (20) ein mit dem Spritzenkörper (10) aus einem Stück geformtes einziges Teil bildet.

2. Vorrichtung nach Anspruch 1, **dadurch gekennzeichnet, daß** die Verbindungswand (22) auf ihrer dem distalen Ende (14B) der Traghülse (14) zugewandten Innenseite eine Vertiefung aufweist, in der das proximale Ende (30A) einer Schubfeder (30) angeordnet ist, deren distales Ende an der Schutzhülse in Anlage ist.

3. Vorrichtung nach Anspruch 1 oder 2, **dadurch gekennzeichnet, daß** die Verbindungswand (22) wenigstens eine Verstärkungsrippe (34) aufweist.

4. Vorrichtung nach einem der Ansprüche 1 bis 3, **dadurch gekennzeichnet, daß** der Ring (20) ein von der Traghülse (14) separates Teil bildet und daß der Ring (20) und die Traghülse (14) jeweils eine Befestigungsschürze (40) aufweisen, wobei diese Schürzen entlang von axialen Flächen, über die der Ring und das Element aneinander befestigt sind, miteinander in Kontakt sind.

5. Vorrichtung nach einem der Ansprüche 1 bis 4, **dadurch gekennzeichnet, daß** der Ring (20) ein von der Traghülse (14) separates Teil bildet und durch eine Befestigungsart, die aus Schweißen, Ineinanderstecken, Aufpressen und Kleben ausgewählt ist, an der Traghülse befestigt ist.

6. Vorrichtung nach einem der Ansprüche 1 bis 5, **dadurch gekennzeichnet, daß** die Haltemittel wenigstens eine Haltelasche (17) umfassen, die den Schlitz (23) durchgreift und die sich auf beiden Seiten der Verbindungswand (22) erstreckt.

7. Vorrichtung nach einem der Ansprüche 1 bis 6, **dadurch gekennzeichnet, daß** die Haltemittel wenigstens eine Haltelasche umfassen, die mit der Schutzhülse fest verbunden ist, und daß sie wenigstens eine Haltefläche (19) für diese Lasche umfaßt, die gegenüber dem Spritzenkörper (10) fest ist, wobei die Haltelasche an der Haltefläche (19) gehalten wird, wenn die Lasche sich in ihrer aktiven Halteausführung befindet, und geeignet ist, bewegt zu werden, um von der Fläche freizukommen.

8. Vorrichtung nach einem der Ansprüche 1 bis 7, ferner umfassend einen Injektionskolben (12), **dadurch gekennzeichnet, daß** der Kolben mit einem Auslöseorgan (13A) fest verbunden ist, das geeignet ist, in Injektionsendstellung des Kolbens das Belasten der Haltemittel außerhalb ihrer aktiven Ausführung auszulösen.

9. Vorrichtung nach Anspruch 8, **dadurch gekennzeichnet, daß** der Ring (20) wenigstens eine Übertragungslasche (37) aufweist, die geeignet ist, durch das Auslöseorgan (13A) bewegt zu werden, um die Haltemittel außerhalb ihrer aktiven Ausführung zu belasten.

## Claims

1. A safe injection device comprising a support sheath (14) having a proximal end (14A) and a distal end (14B), a syringe body (10) secured to said support sheath, and a protection sheath (16) suitable for sliding axially between a retracted position in which it is retracted into an annular space (18) formed between the syringe body (10) and the support sheath (14), and an extended protection position in which it projects beyond the distal end (14B) of the support sheath, the device further comprising retaining means suitable for adopting an active retaining configuration for retaining the protection sheath in the retracted position and, starting from said active configuration, suitable for being urged so as to allow the protection sheath to be extended under drive from thrust means (30), the syringe body is secured to the support sheath via a ring (20) having a coupling wall (22) that extends substantially transversely between the syringe body (10) and the support sheath (14), said wall presenting at least one slot (23), that has a closed outline and through which the retaining means pass so asto be acted upon from the side of the coupling wall (22) that is opposite from the distal end of the support sheath,
**characterized in that** the ring (20) is formed integrally with the syringe body (10).

2. A device according to claim 1, **characterized in that**, in its inside face facing towards the distal end (14B) of the support sheath (14), the coupling wall (22) presents a setback in which the proximal end of a thrust spring (30) is disposed, the distal end of the spring bearing against the protection sheath.

3. A device according to claim 1 or 2, **characterized in that** the coupling wall (22) presents at least one reinforcing rib (34).

4. A device according to any one of claims 1 to 3, **characterized in that** the ring (20) constitutes a part that is distinct from the support sheath (14), and **in that** the ring (20) and said support sheath (14) present respective fastener skirts (40), said skirts coming into contact with each other via axial surfaces whereby the ring and said element are fastened together.

5. A device according to any one of claims 1 to 4, **characterized in that** the ring (20) constitutes a part that is distinct from the support sheath (14) and is fastened to said support sheath by a fastening technique selected from heat-sealing, mutual engagement, a force-fit, and adhesive.

6. A device according to any one of claims 1 to 5, **characterized in that** the retaining means comprise at least one retaining tab (17) that passes through the slot (23) and that extends through the coupling wall (22).

7. A device according to any one of claims 1 to 6, **characterized in that** the retaining means comprise at least one retaining tab that is secured to the protection sheath and **in that** it includes at least one retaining surface (19) for said tab which is stationary relative to the syringe body (10), the retaining tab being retained on the retaining surface (19) when said tab is in its active retaining configuration and being capable of being displaced to escape from said surface.

8. A device according to any one of claims 1 to 7, further comprising an injection piston (12), the device being **characterized in that** the piston is secured to a trigger member (13A) adapted, at the end of the piston injection stroke, to trigger urging the retaining means away from their active configuration.

9. A device according to claim 8, **characterized in that** the ring (20) presents at least one transmission tab (37) adapted to be displaced by the trigger member (13A) to urge the retaining means away from their active configuration.
